Europäisches Patentamt

European Patent Office

Office européen des brevets

(11) Numéro de publication: **0 354 824**
**A2**

## DEMANDE DE BREVET EUROPEEN

(21) Numéro de dépôt: **89402005.6**

(51) Int. Cl.⁵: **A 61 M 5/315**

(22) Date de dépôt: **12.07.89**

(30) Priorité: **01.08.88 FR 8810368**

(43) Date de publication de la demande:
**14.02.90 Bulletin 90/07**

(84) Etats contractants désignés:
**AT BE CH DE ES FR GB GR IT LI LU NL SE**

(71) Demandeur: **Labouze, Joseph**
**53, Boulevard d'Andilly**
**F-95230 Soisy-sous-Montmorency (FR)**

(72) Inventeur: **Labouze, Joseph**
**53 Bld d'Andilly**
**F-95230 Soisy-Sous-Montmorency (FR)**

**Teboul, Pierre**
**4, Avenue Aristide Briand**
**F-95330 Domont (FR)**

(74) Mandataire: **Laget, Jean-Loup et al**
**Cabinet Pierre Loyer 77, rue Boissière**
**F-75116 Paris (FR)**

(54) **Seringue non réutilisable.**

(57) Le piston comporte des nervures perpendiculaires dont le profil en crémaillère 20, 21, coopère avec une butée 19 pour autoriser le mouvement du piston dans un seul sens de sorte que la seringue ne fonctionne qu'une seule fois.

Fig. 1

EP 0 354 824 A2

**Beschreibung**

### Seringue non réutilisable

L'invention concerne une seringue non réutilisable.

L'utilisation des seringues jetables s'est généralisée. Ces seringues sont utilisées une seule fois et sont ensuite jetées. Il arrive cependant, notamment dans la population des drogués, qu'une même seringue soit utilisée par plusieurs personnes, occasionnant ainsi la transmission de maladies et en particulier du sida.

Un but de l'invention est de proposer une seringue qui, après avoir servi une fois, n'est pas réutilisable.

L'invention a pour objet une seringue non réutilisable, du type comportant un corps der seringue et un piston constitué d'un axe longitudinal et de nervures longitudinales perpendiculaires entre elles, caractérisée en ce que les nervures présentent un profil en crémaillère coopérant avec une butée pour autoriser le mouvement du piston dans un seul sens pour chaque nervure.

Selon d'autres caractéristiques de l'invention:
- la butée est disposée dans un anneau obturant en partie le corps de la seringue,
- l'anneau présente une ouverture constituée par un cercle muni de deux fentes dans l'une desquelles est disposée la butée,
- les nervures présentant un profil en crémaillère sont plus larges que les autres et s'insèrent dans les fentes de l'anneau ,
- les autres nervures ne présentent pas de profil en crémaillère et sont de largeur plus faible pour passer dans le cercle ,
- à l'extrémité du profil en crémaillère , les nervures présentent une encoche susceptible d'autoriser, lorsque l'encoche est au droit de l'anneau , le pivotement du piston 1 sur son axe, de telle sorte que, lorsque le piston a reculé pour remplir la seringue, il pivote d'un demi-tour jusqu'à ce que l'autre nervure prenne la place de la première nervure et autorise le mouvement longitudinal du piston pour l'injection,
- le corps de seringue est constitué d'un fût cylindrique et d'un chapeau d'extrémité soudés de façon étanche.

D'autres caractéristiques ressortent de la description qui suit faite avec références au dessin annexé sur lequel on peut voir :

    Figure 1 : une vue en coupe partielle longitudinale axiale d'une seringue selon l'invention;

    Figure 2 : une vue en coupe selon la ligne II de la figure 1.

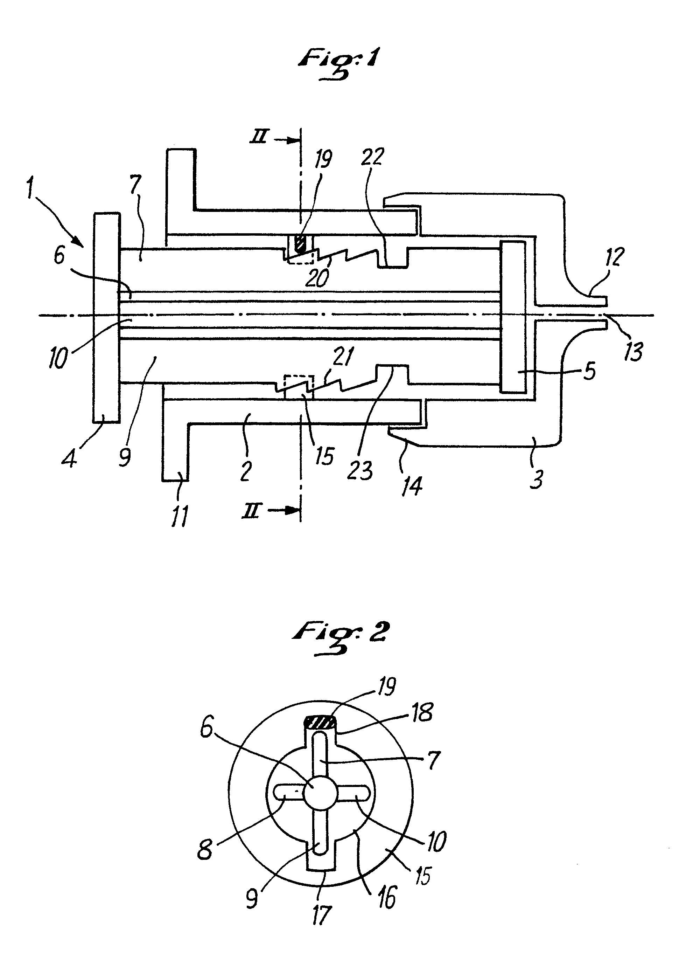

En se reportant à la figure 1, on voit que la seringue selon l'invention se compose essentiellement d'un piston 1 et d'un corps en deux parties assemblées: d'une part un fût cylindrique 2, d'autre part un chapeau d'extrémité 3.

Le piston 1 comporte une collerette de préhension 4 à son extrémité extérieure, un disque 5 coulissant dans le corps, à son extrémité intérieure, et entre les deux un axe 6 cylindrique portant des nervures perpendiculaires 7, 8, 9, 10. Les nervures 8 et 10, opposées l'une à l'autre, ont une faible largeur. Les nervures 7 et 9, opposées diamétralement l'une à l'autre et perpendiculaires aux précédentes, ont une largeur plus grande. Ces nervures sont prévues entre la collerette 4 et le disque 5 pour donner de la rigidité au piston 1.

De plus, et sur une partie de leur bord libre extérieur, les deux nervures les plus larges, 7 et 9, présentent un profil particulier, en crémaillère, dont le rôle sera explicité plus loin.

Le corps de seringue comporte un fût cylindrique 2 muni de son anneau de retenue 11 de type classique, et un chapeau d'extrémité 3 comportant lui-même une partie cylindrique s'appliquant sur l'extrémité du fût 2, et un opercule d'extrémité muni d'un bec 12 pour fixer l'aiguille et présentant un passage 13 pour le produit à injecter. Le chapeau d'extrémité 3 peut aussi porter une aiguille incorporée. Le chapeau 3 est muni d'un anneau 14 enveloppant l'extrémité du fût 2 et susceptible de recevoir un anneau de soudure pour assurer la solidarisation étanche du fût et du chapeau.

A l'intérieur du fût 2 est disposé un anneau 15 qui est représenté sur la figure 2. Cet anneau 15 èst fixé au fût, éventuellement par collage. Son diamètre extérieur correspond au diamètre intérieur du fût. Sa partie centrale présente une ouverture constituée par un cercle 16 muni de deux fentes opposées 17 et 18. Dans l'une de ces fentes, 18 par exemple, est disposée une butée 19 destinée à coopérer avec les profils des nervures 7 et 9.

Le cercle 16 est de diamètre suffisant pour que les nervures les moins larges 8 et 10 puissent y passer sans frottement. En revanche, les nervures les plus larges 7 et 9 ne peuvent que s'insérer dans les fentes opposées 17 et 18. De cette manière le piston est orienté et guidé dans son déplacement longitudinal, et la butée 19 peut coopérer avec le profil des nervures 7 et 9.

La nervure 7 présente un profil en crémaillère 20 qui permet au piston 1 de reculer pour permettre le remplissage de la seringue, mais qui ne permet pas au piston d'avancer pour procéder à l'injection. En revanche, la nervure 9 présente un profil en crémaillière 21 qui permet au piston d'avancer pour procéder à l'injection, mais qui ne lui permet pas de reculer pour remplir de nouveau la seringue.

A l'extrémité du profil en crémaillère, du côté du disque 5, chacune des nervures 7 et 9 présente une encoche, respectivement 22 et 23. Lorsque cette encoche se trouve au droit de l'anneau 15, le piston 1 peut pivoter sur son axe longitudinal. Cette faculté de pivotement est utilisée de la manière suivante :

Le piston 1 étant en place dans le corps de seringue, la nervure 7 étant logée dans la fente 18 de l'anneau 15, le profil 20 étant en appui sur la butée 19, on retire le piston 1 pour remplir la seringue. Les crans du profil 20 en crémaillère franchissent l'un après l'autre la butée 19 par déformation plastique, tout en empêchant l'avancée du piston pour une injection. Lorsque l'encoche 22 arrive au droit de l'anneau 15, le piston 1 peut pivoter sur lui-même d'un demi-tour, jusqu'à ce que la nervure 7 se loge dans la fente 17 et la nervure 9 dans la fente 18.

Le piston 1 peut alors avancer pour assurer une injection. Les crans du profil 1 en crémaillère franchissent l'un après l'autre la butée 19 par déformation plastique, tout en interdisant le retour du piston 1 vers l'arrière. Lorsque l'injection est terminée, le piston est bloqué dans sa position finale. Il ne peut reculer car les crans du profil 21 et la butée 19 l'en empêchent. Il ne peut pivoter sur son axe car les nervures 7 et 9 sont dans les fentes 17, 18 de l'anneau 15 qui les maintiennent. La seringue n'est donc pas réutilisable.

La seringue selon l'invention ne peut être montée comme une seringue habituelle, c'est pourquoi elle est en plusieurs pièces qu'il faut assembler dans un ordre précis.

Tout d'abord, on insère l'anneau 15 dans le fût 2 et on le met en place. On le fixe, par collage par exemple.

On met en place le piston 1 auquel il manque une extrémité. Si l'on n'a pas encore fixé le disque 5, on met le piston en place par l'extrémité du fût correspondant à l'anneau de retenue 11. Si l'on n'a pas encore fixé la collerette de préhension 4, on met le piston en place par l'extrémité du fût correspondant au chapeau 3.

On assemble ensuite l'extrémité manquante du piston 1, par soudage par ultrasons par exemple.

On met ensuite en place le chapeau d'extrémité 3, dont l'anneau 14 enveloppe l'extrémité du fût 2. On peut assurer la solidarisation du fût 2 et de l'anneau 14 par soudage par ultrasons par exemple. La liaison entre le chapeau 3 et le fût 2 doit bien entendu être étanche. La seringue est alors prête à fonctionner une seule fois, et elle n'est pas réutilisable.

Eventuellement, et pour accentuer le caractère non réutilisable de la seringue, on peut fragiliser le piston 1, dans un plan perpendiculaire à son axe et disposé entre la collerette de préhension 4 et le profil en crémaillère 21. A cet effet, on peut prévoir des lignes de moindre résistance dans les nervures 7 à 10 et un point de faiblesse sur l'axe 6. De cette façon, après usage de la seringue, si on essaie de la réutiliser en tirant fortement sur le piston 1, celui-ci se rompt, ce qui confirme l'impossibilité de réutiliser la seringue.

## Revendications

1. Seringue non réutilisable, du type comportant un corps de seringue et un piston constitué d'un axe longitudinal et de nervures perpendiculaires, caractérisée en ce que les nervures (7, 9) présentent un profil en crémaillère (20, 21) coopérant avec une butée (19) pour autoriser le mouvement du piston (1) dans un seul sens pour chaque nervure.

2. Seringue selon la revendication 1, caractérisée en ce que la butée (19) est disposée dans un anneau (15) obturant en partie le corps de la seringue.

3. Seringue selon la revendication 2, caractérisée en ce que l'anneau (15) présente une ouverture constituée par un cercle (16) muni de deux fentes (17, 18) dans l'une (18) desquelles est disposée la butée.

4. Seringue selon la revendication 3, caractérisée en ce que les nervures (7, 9) présentant un profil en crémaillère sont plus larges que les autres et s'insèrent dans les fentes (17, 18) de l'anneau (15).

5. Seringue selon la revendication 4, caractérisée en ce que les autres nervures (8, 10) ne présentent pas de profil en crémaillère et sont de largeur plus faible pour passer dans le cercle (16).

6. Seringue selon la revendication 5, caractérisée en ce qu'à l'extrémité du profil en crémaillère (20, 21), les nervures (7, 9) présentent une encoche (22, 23) susceptible d'autoriser, lorsque l'encoche est au droit de l'anneau (15), le pivotement du piston 1 sur son axe, de telle sorte que, lorsque le piston a reculé pour remplir la seringue, il pivote d'un demi-tour jusqu'à ce que l'autre nervure (9) prenne la place de la première nervure (7) et autorise le mouvement longitudinal du piston (1) pour l'injection.

7. Seringue selon la revendication 1, caractérisée en ce que le corps de seringue est constitué d'un fût (2) cylindrique et d'un chapeau d'extrémité (3) soudés de façon étanche.

Fig. 1

Fig. 2